(19) Europäisches Patentamt
European Patent Office
Office européen des brevets

(11) Veröffentlichungsnummer: **0 325 983**
**A2**

(12) # EUROPÄISCHE PATENTANMELDUNG

(21) Anmeldenummer: **89100672.8**

(22) Anmeldetag: **17.01.89**

(51) Int. Cl.4: **C07C 103/76 , C07C 131/00 , C07C 121/52 , C07C 161/03 , C07C 109/10 , A01N 37/40 , A01N 37/52 , A01N 37/34**

Patentanspruch für folgenden Vertragsstaat: ES

(30) Priorität: **26.01.88 DE 3802175**

(43) Veröffentlichungstag der Anmeldung: **02.08.89 Patentblatt 89/31**

(84) Benannte Vertragsstaaten: **CH DE ES FR GB IT LI**

(71) Anmelder: **HOECHST AKTIENGESELLSCHAFT**
**Postfach 80 03 20**
**D-6230 Frankfurt am Main 80(DE)**

(72) Erfinder: **Kern, Manfred, Dr.**
**Im Traminer Weg 8**
**D-6501 Lörzweiler(DE)**
Erfinder: **Knauf, Werner, Dr.**
**Im Kirschgarten 24**
**D-6239 Eppstein/Taunus(DE)**
Erfinder: **Matterstock, Karl, Dr.**
**Lessingstrasse 38**
**D-6238 Hofheim am Taunus(DE)**
Erfinder: **Sachse, Burkhard, Dr.**
**An der Ziegelei 30**
**D-6233 Kelkheim Taunus(DE)**
Erfinder: **Schmidt, Ernst, Dr.**
**Homburger Strasse 9**
**D-6238 Hofheim am Taunus(DE)**
Erfinder: **Schuck, Ernst, Dr.**
**Kleine Hecke 20**
**D-8751 Leidersbach(DE)**
Erfinder: **Waltersdorfer, Anna, Dr.**
**Rauenthaler Weg 28**
**D-6000 Frankfurt am Main 71(DE)**
Erfinder: **Wicke, Heinrich, Dr.**
**Schillerstrasse 3**
**D-6239 Eppstein/Taunus(DE)**

(54) **N-Phenylbenzamide und N-Phenylbenzamidoxime, Verfahren zu ihrer Herstellung, sie enthaltende Mittel und ihre Verwendung als Schädlingsbekämpfungsmittel.**

(57) Verbindungen der allgemeinen Formel I

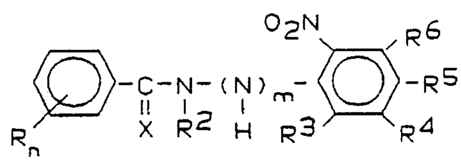

(I),

worin
X O, S oder $NOR^1$,
R Wasserstoff, Halogen, CN, SCN, $NO_2$, OH, Carbamoyl, Alkyl, Alkoxy, Thioalkyl, die ganz oder teilweise durch Halogen substituiert sein können, Phenyl, Thiophenyl oder Phenoxy, die durch 1 oder 2 Halogenatome oder eine $CF_3$-Gruppe substituiert sein können, oder Acyl,
$R^1$ Wasserstoff, Alkyl, Alkenyl, Alkinyl, die ganz oder teilweise durch Halogen substituiert sein könne, Acyl, Cyanomethyl,
$R^2$ Wasserstoff, Alkyl oder $S\text{-}CCl_3$,

EP 0 325 983 A2

$R^3$,$R^5$ jeweils unabhängig voneinander $NO_2$, Halogen, CN, COOH, $CONH_2$, Alkyl oder Alkoxy, die ganz oder teilweise durch Halogen substituiert sein können, Alkoxycarbonyl, $CONR^7R^8$,

$R^4$ Wasserstoff oder Halogen,

$R^6$ Wasserstoff, Halogen, Alkoxy oder Phenoxy,

$R^7$,$R^8$ Alkyl,

m 0 oder 1 und

n 0 - 5

bedeuten, mit der Maßgabe, daß im Fall X = $NOR^1$ m = 0 sein muß, sowie deren in der Landwirtschaft einsetzbaren Salze besitzen vorteilhafte insektizide, akarizide, fungizide oder nematozide Eigenschaften.
Ferner werden Verfahren zur Herstellung von Verbindungen der Formel I beschrieben.

# N-Phenylbenzamide und N-Phenylbenzamidoxime, Verfahren zu ihrer Herstellung, sie enthaltende Mittel und ihre Verwendung als Schädlingsbekämpfungsmittel

Es ist bekannt, daß gewisse substituierte Benzanilide, wie sie im Journal of med. Chemistry 12, 957 (1969) beschrieben sind, antibakterielle Eigenschaften aufweisen. Es wurden nun neue N-Phenylbenzamide und N-Phenylbenzamidoxime gefunden, die vorteilhafte insektizide, fungizide, akarizide oder nematozide Wirkungen besitzen.

Gegenstand der vorliegenden Erfindung sind daher Verbindungen der Formel I

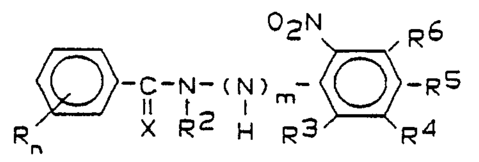

( I ),

worin

X O, S oder $NOR^1$,

R Wasserstoff, Halogen, CN, SCN, $NO_2$, OH, Carbamoyl, $(C_1-C_4)$ Alkyl, $(C_1-C_4)$-Alkoxy, $(C_1-C_4)$-Thioalkyl, die ganz oder teilweise durch Halogen substituiert sein können, Phenyl, Thiophenyl oder Phenoxy, die durch 1 oder 2 Halogenatome oder eine $CF_3$-Gruppe substituiert sein können, oder $(C_1-C_4)$-Acyl,

$R^1$ Wasserstoff, $(C_1-C_4)$-Alkyl, $(C_2-C_4)$-Alkenyl, $(C_2-C_4)$-Alkinyl, die ganz oder teilweise durch Halogen substituiert sein können, $(C_1-C_4)$-Acyl, Cyanomethyl,

$R^2$ Wasserstoff, $(C_1-C_4)$-Alkyl oder $S-CCl_3$,

$R^3$,$R^5$ jeweils unabhängig voneinander $NO_2$, Halogen, CN, COOH, $CONH_2$, $(C_1-C_4)$-Alkyl oder $(C_1-C_4)$-Alkoxy, die ganz oder teilweise durch Halogen substituiert sein können, $(C_1-C_4)$-Alkoxycarbonyl, $CONR^7R^8$,

$R^4$ Wasserstoff oder Halogen,

$R^6$ Wasserstoff, Halogen, $(C_1-C_4)$-Alkoxy oder Phenoxy,

$R^7$,$R^8$ $(C_1-C_4)$-Alkyl,

m 0 oder 1 und

n 0 - 5

bedeuten, mit der Maßgabe, daß im Fall $X = NOR^1$ $m = 0$ sein muß, sowie deren in der Landwirtschaft einsetzbaren Salze.

Dabei bedeutet Alkyl sowohl geradkettiges als auch verzweigtes Alkyl und Halogen, bevorzugt F und Cl.

Bevorzugt unter den Verbindungen der Formel (I) sind solche, bei denen R = Halogen oder $CF_3$, n = 1 oder 2, X = Sauerstoff oder $NOR^1$, $R^1 = (C_1-C_4)$-AlkYl, $R^2$ = H, $R^3 = NO_2$ oder $CF_3$, $R^4$ = H, $R^5 = NO_2$, $CF_3$ oder CN, $R^6$ = Wasserstoff oder Chlor und m = 0 bedeuten, sowie deren in den Landwirtschaft einsetzbaren Salze.

Besonders bevorzugt unter den Verbindungen der Formel (I) sind: N-(2,6-Dinitro-4-trifluormethyl-phenyl)-4-trifluormethyl-methylbenzamidoxim, N-(3-Chlor-2,6-dinitro-4-trifluormethylphenyl)-4-trifluormethyl-methylbenzamidoxim, N-(2,4-Dinitro-6-trifluormethyl-phenyl)-4-trifluormethylmethylbenzamidoxim, N-(3-Chlor-2,6-dinitro-4-trifluormethylphenyl)-4-trifluormethyl-allylbenzamidoxim, N-(4-Cyano-2,6-dinitro-phenyl)-4-trifluormethyl-methylbenzamidoxim, N-(3-Chlor-4-cyano-2,6-dinitro-phenyl)-4-trifluormethylmethylbenzamidoxim, N-(3-Chlor-2,6-dinitro-4-trifluormethylphenyl)-3-trifluormethyl-methylbenzamidoxim, N-(2,4-Dinitro-6-trifluormethyl-phenyl)-3-trifluormethyl-methylbenzamidoxim, N-(3-Chlor-2,6-dinitro-4-trifluormethylphenyl)-4-chlor-methylbenzamidoxim, N-(3-Chlor-2,6-dinitro-4-trifluormethyl-phenyl)-4-methyl-methylbenzamidoxim, N-(2,6-Dinitro-4-trifluormethylphenyl)-4-trifluormethylbenzamid, N-(3-Chlor-2,6-dinitro-4-trifluormethylphenyl)-4-trifluormethylbenzamid, N-(3-Chlor-2,6-dinitro-4-trifluormethylphenyl)-4-chlorbenzamid, N-(3-Chlor-2,6-dinitro-4-trifluormethylphenyl)-3-chlorbenzamid, N-(2,6-Dinitro-4-trifluormethylphenyl)-4-chlorbenzamid, N-(2,6-Dinitro-4-trifluormethylphenyl)-3-chlorbenzamid, N-(2,6-Dinitro-4-trifluormethylphenyl)-3,4-dichlorbenzamid, N-(3-Chlor-2,6-dinitro-4-trifluormethylphenyl)-3,4-dichlorbenzamid, N-(2,6-Dinitro-4-trifluormethylphenyl)-3-trifluormethylbenzamid, N-(3-Chlor-2,6-dinitro-4-trifluormethylphenyl)-3-trifluormethylbenzamid, N-(2,6-Dinitro-4-trifluormethylphenyl)-2-chlor-4-trifluormethylbenzamid, N-(3-Chlor-2,6-dinitro-4-trifluormethylphenyl)-2-chlor-4-trifluormethylbenzamid, sowie deren in der Landwirtschaft einsetzbaren Salze.

Die Erfindung umfaßt auch die Salze der Verbindungen der Formel I, welche durch Substitution von $R^2$ durch entsprechende Metallionen oder Ammonium gebildet werden. Im Falle der Hydrazide (m = 1) erfolgt

die Salzbildung durch Deprotonierung am aciden Stickstoffatom, das dem $NO_2$-substituierten Phenylkern direkt benachbart ist.

Als Salze der Verbindungen der Formel I kommen die in der Landwirtschaft einsetzbaren Salze in Frage, insbesondere die Alkali-, Erdalkali-, Ammonium- oder ein- bis vierfach durch organische Reste (wie Alkyl, Hydroxyalkyl) substituierte Ammoniumsalze.

Gegenstand der Erfindung sind ferner Verfahren zur Herstellung der Verbindungen der Formel (I), dadurch gekennzeichnet, daß man

a) eine Verbindung der Formel (II)

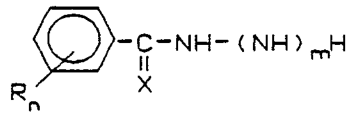

(II),

worin R, X, n und m die Bedeutungen wie in Formel I besitzen,
mit einer Verbindung der Formel (III)

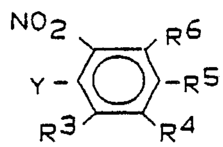

(III),

worin $R^3$, $R^4$, $R^5$ und $R^6$ die Bedeutungen wie in Formel I besitzen und Y Halogen bedeutet, in Gegenwart einer Base umsetzt, oder

b) eine Verbindung der Formel (IV)

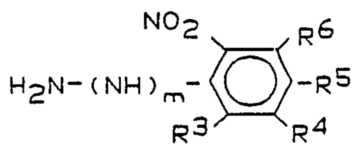

(IV),

worin m, $R^3$, $R^4$, $R^5$ und $R^6$ die Bedeutungen wie in Formel I besitzen,
mit einer Verbindung der Formel (V)

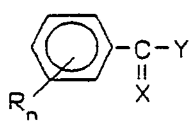

(V),

worin R, n und X die Bedeutungen wie in Formel I besitzen und Y Halogen bedeutet, umsetzt.

Das prinzipielle Herstellungsverfahren für die als Vorstufen benötigten Verbindungen (II) ist in Chem. Rev. 62 (1962) 155 - 183 beschrieben.

Die Verbindungen der Formel III sind zu einem großen Teil im Handel erhältlich oder lassen sich nach literaturbekannten Methoden leicht herstellen.

Die Verbindungen der Formel IV erhält man durch Umsetzung von Verbindungen der Formel III mit Ammoniak oder Hydrazin.

Verbindungen der Formel V sind in Chem. Ber. 18 (1985) 1189 beschrieben.

Die Salze der Verbindungen der Formel I erhält man nach für den Fachmann geläufigen Methoden, wie z. B. Umsetzung mit Alkali- oder Erdalkalihydroxiden. Ein Baseüberschuß während der Reaktion zur Herstellung der Verbindungen der Formel I führt direkt zu den entsprechenden Salzen.

Vorzugsweise werden die Verbindungen in Gegenwart einer Base und eines oder mehrerer Lösungs- oder Verdünnungsmittel hergestellt.

Geeignete inerte Lösungsmittel sind aliphatische oder aromatische Kohlenwasserstoffe wie Benzol, Toluol,

Xylol und Petrolether. Halogenierte Kohlenwasserstoffe wie Chlorbenzol, Methylenchlorid, Ethylenchlorid, Chloroform, Tetrachlorkohlenstoff oder Tetrachlorethylen, Ether wie Dialkylether, Anisol, Tetrahydrofuran; Nitrile wie Acetonitril, Propionitril, N,N-dialkylierte Amide wie Dimethylformamid, Dimethylsulfoxid und Ketone wie z. B. Aceton oder Methylethylketon.

Geeignete Basen sind die dem Fachmann geläufigen Verbindungen wie (Erd)alkalimetallcarbonate wie Kaliumcarbonat, Ca-carbonat oder (Erd)alkalimetallhydroxide wie Kaliumhydroxid, Natriumhydroxid, Calciumhydroxid oder auch Natriumhydrid, Butyllithium, Kalium-tert.-butylat.

Die Temperatur bei der Umsetzung hängt von der Auswahl der Reaktionspartner, des Lösungs- oder Verdünnungsmittels und der Base ab.

Im allgemeinen liegt die Reaktionstemperatur im Intervall von -30° C bis 130° C, bevorzugt bei Temperaturen von -20° C bis 80° C.

Die Verbindungen II und III bzw. IV und V werden in etwa stöchiometrischen Mengen eingesetzt, wobei eine Komponente auch in einem Überschuß bis zu 10 % angewandt werden kann.

Das säurebindende Mittel wird in mindestens stöchiometrischer Menge, meist jedoch im Überschuß eingesetzt. Die Aufarbeitung erfolgt in üblicher Weise bei mit Wasser nicht mischbaren Lösungsmitteln durch Ausschütteln mit Wasser, Trocknen der organischen Phase und Eindampfen. Bei mit Wasser mischbaren Lösungsmitteln durch Verdünnen mit Wasser und Extrahieren des Produkts mit einem nicht wassermischbaren Lösungsmittel.

Die Verbindungen der Formel I fallen kristallin oder als nicht destillierbare Öle an. Zur weiteren Reinigung kristallisiert man aus einem geeigneten Lösungsmittel um oder man reinigt die Produkte durch Chromatographie über Kieselgel.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytopathogener Pilze wie z. B. Piricularia oryzae, Venturia inaequalis, Cercospora beticola, Echte Mehltauarten, Fusariumarten, Plasmopara viticola, Pseudoperonospora cubensis, verschiedene Rostpilze und Pseudocercosporella herpotrichoides. Besonders gut werden Benzimidazol- und Dicarboximid-sensible und -resistente Botrytis cinerea-Stämme erfaßt.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr-und Schneidölen.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z. B. folgende Produkte zu nennen:

Imazalil, Prochloraz, Fenapanil, SSF 105, Triflumizol, PP 969, Flutriafol, BAY-MEB 6401, Propiconazol, Etaconazol, Diclobutrazol, Bitertanol, Triadimefon, Triadimenol, Fluotrimazol, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol,

Bupirimate, Rabenzazole, Tricyclazole, Fluobenzimine, Pyroxyfur, NK-483, PP-389, Pyroquilon, Hymexazole, Fenitriopan, UHF-8227, Cymoxanil, Dichlorunanid, Captafol, Captan, Folpet, Tolylfluanid, Chlorothalonil, Etridiazol, Iprodione (Formel II), Procymidon, Vinclozolin, Metomeclan, Myclozolin, Dichlozolinate, Fluorimide, Drazoxolan, Chinomethionate, Nitrothalisopropyl, Dithianon, Dinocap, Binapacryl,

Fentinacetate, Fentinhydroxide, Carboxin, Oxycarboxin, Pyracarbolid, Methfuroxam, Fenfura, Furmecyclox, Benodanil, Mebenil, Mepronil, Flutalanil, Fuberidazole, Thiabendazole, Carbendazim, Benomyl, Thiofante, Thiofanatemethyl, CGD-94340 F, IKF-1216,

Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cuoxychlorid, Oxine-Cu, Cu-oxide, Schwefel, Fosetylaluminium,

Natrium-dodecylbenzolsulfonat,
Natrium-dodecylsulfat,
Natrium-C13/C15-alkoholethersulfonat,
Natrium-cetostearylphosphatester,
Dioctyl-natriumsulfosuccinat,
Natrium-isopropylnaphthalinsulfonat,
Natrium-methylenbisnaphthalinsulfonat,

Cetyl-trimethyl-ammoniumchlorid,
Salze von langkettigen primären, sekundären oder tertiären Aminen, Alkyl-propylenamine, Laurylpyridinium-bromid, ethoxylierte quaternierte Fettamine, Alkyl-dimethyl-benzylammoniumchlorid und 1 Hydroxyethyl-2-alkyl-imidazolin.

Die oben genannten Kombinationspartner stellen bekannte Wirkstoffe dar, die zum großen Teil in CH. R. Worthing, U.S.B. Walker, The Pesticide Manual, 7. Auflage (1983), British Crop Protection Council beschrieben sind.

Die Wirkstoffe eignen sich ferner bei guter Pflanzenverträgliohkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:

Aus der Ordnung der Acarina z.B. Acarus siro, Agras spp., Ornithrodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.
Aus der Ordnung der Isopoda, z.B. Oniscus asselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregarai.
Aus der Ordnung der Dermaptera z.B. Forficula auricularia.
Aus der Ordnung des Isoptera z.B. Reticulitermes spp.
Aus der Ordnung der Anoplura z.B. Phylloera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporarium, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Naphotettix cincticeps, Lecanium corni, Saissetia oleae, Laodelphax striatellus, Nilaparvata lugens, Aonidiella auroantii, Apidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cocoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrhynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliophora erythrocephala, Lucilia spp., Chrysomyia spp., Cuterebra spp., Gastrophilus spp., Hypobosca spp., Stomoxys spp., Oestrus spp. Hypoderma spp., Tabanus spp., Tannia spp., Bibio

hortulanus, Oscinella frit, Phorbia spp., Pegomyia hyoscyami, Ceratitis capitata, Dacus oleae, Tripula paludosa.

Aus der Ordnung der Siphonaptera z.B. Xenopsylla cheopsis, Ceratophyllus spp.

Aus der Ordnung der Arachnida z.B. Scorpio maurus, Latrodectus mactans.

Aus der Klasse der Helminthen z.B. Haemonchus, Trichostrongulus, Ostertagia. Cooperia, Chabertia, Strongyloides, Oesophagostomum, Hyostrongulus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphinema.

Aus der Klasse der Gastropoda z.B. Deroceras spp., Arion spp., Lymnaea spp., Galba spp., Succinea spp., Biophalaria spp., Bulinus spp., Oncomelania spp.

Aus der Klasse der Bivalva z.B. Dreissena spp.

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemischphysikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel,

Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a. Bevorzugte Mischungspartner sind

1. aus der Gruppe der Phosphorverbindungen

Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos, O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. aus der Gruppe der Carbamate

Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cu-menylbutyryl(methyl)-carbamate, Oxamyl, Pirimicarb, Propoxur, Thiodicarb, Thiofanox, Ethyl 4,6,9-triaza-4-benzyl-6,l0-dimethyl-8-oxa-7-oxo-5,11-dithia-g-dodecenoate (OK 135), 1-methylthio(ethylideneamino)N-methyl-N-(morpholinothio)-carbamate (UC 51717);

3. aus der Gruppe der Carbonsäureester

Allethrin, Alphametrin, 5-Benzyl-3-furylmethyl-(E)-(1R)-cis-2,2-di-methyl-3-(2-oxothiolan-3-ylidenemethyl)cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin;

4. aus der Gruppe der Amidine

Amitraz, Chlordimeform;

5. aus der Gruppe der Zinnverbindungen

Cyhexatin, Fenbutatinoxide;

6. Sonstige

Abamectin, Bacillus thuringiensis, Bensultap, Binapacryl, Bromopropylate, Buprofezin, Camphechlor, Cartap, Chlorobenzilate, Chlcrfluazuron, 2-(4-(Chlorphenyl)-4,5-di-phenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenylcarbamoyl)-2-chlorbenzcarboximidsäureethylester, DDT, Dicofol, N-(N-(3,5- Dichlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)-propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2 Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (Z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Nachfolgende Beispiele dienen zur Erläuterung der Erfindung.

**A. Formulierungsbeispiele**

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die

Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

## B. Chemische Beispiele

### Beispiel 1

N-(2,4-Dinitro-6-trifluormethylphenyl)-2-acetoxybenzamid

Zu einer Lösung von 4,5 g 2-Acetoxybenzamid in 150 ml Tetrahydrofuran tropft man unter Rühren bei 0 - 5 °C eine Lösung von 6,7 g 2-Chlor-3,5-dinitrobenzotrifluorid in 50 ml Tetrahydrofuran. Während des Zutropfens gibt man portionsweise zum Reaktionsgemische 4 g fein gepulvertes, 88 %iges Kaliumhydroxid. Man rührt das Reaktionsgemisch zwei Stunden bei 0 °C und 12 Stunden bei Raumtemperatur, gießt in zwei Liter Wasser und säuert mit konz. Salzsäure auf pH 2 an. Nach einer Stunde saugt man vom Niederschlag ab und wäscht mit Wasser. Das Reaktionsprodukt fällt in quantitativer Ausbeute in farblosen Kristallen an. Fp.: 157 °C.

Die Verbindungen der allgemeinen Formel I der Tabellen 1 bis 5 lassen sich auf entsprechende Weise herstellen.

### Beispiel 2

N-(3-Chlor-2,6-dinitro-4-trifluormethylphenyl)-4-trifluormethyl-methylbenzamidoxim

Zu einer Suspension von 3,3 g (110 mmol) 80 %igem Natriumhydrid in 250 ml Tetrahydrofuran tropft man unter Rühren bei 0 °C eine Mischung aus 10,9 g (50 mmol) 4-Trifluormethyl-methylbenzamidoxim und 15,3 g (50 mmol) 2,4-Dichlor-3,5-dinitrobenzotrifluorid in 150 ml Tetrahydrofuran und hält das Reaktionsgemisch für 6 h auf 20 °C. Nach Zugabe von 8 ml Ethanol wird die Reaktionslösung in ein Gemisch aus 300 ml 0.5 N wäßriger Salzsäure und 300 ml Dichlormethan gegossen, die Phasen getrennt und die wäßrige Phase noch zweimal mit jeweils 100 ml Dichlormethan extrahiert. Die vereinigten organischen Phasen trocknet man über Natriumsulfat und engt sie bei 40 °C/20 mm ein.

Chromatographie des Rückstands an einer Kieselgelsäule mit Dichlormethan/Petrolether im Verhältnis 1 : 1 liefert 16,1 g der obengenannten Verbindung (66 % d. Th.) in Form gelblicher Kristalle. Fp.: 118 °C.

Die Verbindungen der allgemeinen Formel I der Tabelle 6 lassen sich auf analoge Weise herstellen.

**Tabelle 6** Fortsetzung

| Bsp. Nr. | $R_n$ | $R^1$ | $R^2$ | $R^3$ | $R^4$ | $R^5$ | $R^6$ | Fp. |
|---|---|---|---|---|---|---|---|---|
| 155 | 2-Cl,4-$CF_3$ | Me | H | $NO_2$ | H | $CF_3$ | H | 76° |
| 156 | 2-Cl,4-$CF_3$ | Me | H | $NO_2$ | H | $CF_3$ | Cl | 116° |
| 157 | 2-Cl,4-$CF_3$ | Me | H | $CF_3$ | H | $NO_2$ | H | |
| 158 | 2-Cl,4-$CF_3$ | Me | H | $NO_2$ | H | CN | H | |
| 159 | 4-$NO_2$ | Me | H | $NO_2$ | H | $CF_3$ | Cl | |
| 160 | 4-CN | Me | H | $NO_2$ | H | $CF_3$ | Cl | 193° |
| 161 | 4-Me | Me | H | $NO_2$ | H | $CF_3$ | H | 117° |
| 162 | 4-Me | Me | H | $NO_2$ | H | $CF_3$ | Cl | 136° |
| 163 | 4-Me | Me | H | $CF_3$ | H | $NO_2$ | H | |
| 164 | 4-Me | Me | H | $NO_2$ | H | $CO_2Et$ | H | 136° |
| 165 | 4-t-But | Me | H | $NO_2$ | H | $CF_3$ | Cl | Öl |
| 166 | 4-$OCF_3$ | Me | H | $NO_2$ | H | $CF_3$ | H | 108° |
| 167 | 4-$OCF_3$ | Me | H | $NO_2$ | H | $CF_3$ | Cl | 88° |
| 168 | 4-$OCF_3$ | Me | H | $CF_3$ | H | $NO_2$ | H | |
| 169 | 4-OMe | Me | H | $NO_2$ | H | $CF_3$ | H | 121° |
| 170 | 4-OMe | Me | H | $NO_2$ | H | $CF_3$ | Cl | 120° |
| 171 | 4-OMe | Me | H | $CF_3$ | H | $NO_2$ | H | |
| 172 | 4-SMe | Me | H | $NO_2$ | H | $CF_3$ | Cl | |
| 173 | 4-$SO_2Me$ | Me | H | $NO_2$ | H | $CF_3$ | Cl | |
| 174 | 4-$SO_2Me$ | Me | H | $CF_3$ | H | $NO_2$ | H | |
| 175 | 3-Cl | Me | H | $CF_3$ | H | $NO_2$ | H | Öl |
| 176 | 4-Cl | $-CF_2CHFCF_3$ | H | $NO_2$ | H | $CF_3$ | H | Öl |
| 177 | 4-Cl | $-COCH_3$ | H | $NO_2$ | H | $CF_3$ | H | 110° |
| 178 | 4-F | $-CH_2CH_2CH_3$ | H | $NO_2$ | H | $CF_3$ | Cl | 74° |
| 179 | 2-F | Me | H | $NO_2$ | H | $CF_3$ | H | Öl |

Me = Methyl
Et = Ethyl

**Tabelle 6** Fortsetzung

| Bsp. Nr. | $R_n$ | $R^1$ | $R^2$ | $R^3$ | $R^4$ | $R^5$ | $R^6$ | Fp. |
|---|---|---|---|---|---|---|---|---|
| 180 | 4-Br | Me | H | $NO_2$ | H | $CF_3$ | H | 129° |
| 181 | 4-Br | Me | H | $NO_2$ | H | $CF_3$ | Cl | 141° |
| 182 | 4-Br | Me | H | $CF_3$ | H | $NO_2$ | H | 113° |
| 183 | 4-I | Me | H | $NO_2$ | H | $CF_3$ | H | 123° |
| 184 | 4-I | Me | H | $NO_2$ | H | $CF_3$ | Cl | 110° |
| 185 | 4-I | Me | H | $CF_3$ | H | $NO_2$ | H | 122° |
| 186 | 2,4-$F_2$ | Me | H | $NO_2$ | H | $CF_3$ | H | 71° |
| 187 | 2,4-$F_2$ | Me | H | $NO_2$ | H | $CF_3$ | Cl | 88° |
| 188 | 2,4-$F_2$ | Me | H | $CF_3$ | H | $NO_2$ | H | Öl |
| 189 | 2,6-$F_2$ | $-CH_2-C{\equiv}CH$ | H | $NO_2$ | H | $CF_3$ | H | 114° |
| 190 | 2-Cl,4-F | Me | H | $NO_2$ | H | $CF_3$ | H | 86° |
| 191 | 2-Cl,4-F | Me | H | $NO_2$ | H | $CF_3$ | Cl | 102° |
| 192 | 4-$CF_2Cl$ | Me | H | $NO_2$ | H | $CF_3$ | H | 109° |
| 193 | 4-$CF_2Cl$ | Me | H | $NO_2$ | H | $CF_3$ | Cl | 64° |
| 194 | 4-t-But | Me | H | $NO_2$ | H | $CF_3$ | H | Öl |
| 195 | 3,4-$(OC_2H_5)_2$ | Me | H | $NO_2$ | H | $CF_3$ | Cl | 105° |

Me = Methyl
Et = Ethyl

## C. Biologische Beispiele

### Beispiel 1

Weinsämlinge der Sorten "Riesling/Ehrenfelder" wurden ca. 6 Wochen nach der Aussaat mit wäßrigen Suspensionen der beanspruchten Verbindungen tropfnaß behandelt. Die Anwendungskonzentrationen betrugen 500, 250 und 125 mg Wirkstoff bzw. 500 und 250 mg Wirkstoff pro Liter Spritzbrühe.

Nach dem Antrocknen des Spritzbelages wurden die Pflanzen mit einer Zoosporangiensuspension von Plasmopara viticola inokuliert und tropfnaß in eine Klimakammer bei einer Temperatur von ca 23° C und einer Luftfeuchtigkeit von ca. 80 - 90 % gebracht.

Nach einer Inkubationszeit von 7 Tagen wurden die Pflanzen über Nacht in die Klimakammer gestellt und die Krankheit zum Ausbruch gebracht. Anschließend erfolgt die Befallsauswertung. Der Befallsgrad wurde in % befallener Blattfläche im Vergleich zu den unbehandelten, infizierten Kontrollpflanzen ausgedrückt und ist in Tabellen 7 und 8 wiedergegeben.

Tabelle 1

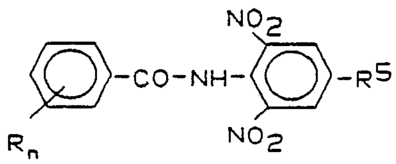

( I )

| Bsp.-Nr. | $R_n$ | $R^5$ | Fp |
|---|---|---|---|
| 3 | 4-$CF_3$ | $CF_3$ | 177° |
| 4 | 2,6-$F_2$ | $CF_3$ | 202° |
| 5 | 4-Cl | $CF_3$ | 116° |
| 6 | 2-F | $CF_3$ | 150° |
| 7 | 2,6-$Cl_2$ | $CF_3$ | 275° |
| 8 | 2,4-$Cl_2$ | $CF_3$ | 217° |
| 9 | 4-$CF_3O$ | $CF_3$ | 129° |
| 10 | 4-J | $CF_3$ | 164° |
| 11 | 4-$CF_3$,2-Cl | $CF_3$ | 227° |
| 12 | 4-$CF_3$ | $CCl_3$ | 90° |
| 13 | 4-Cl | $CCl_3$ | 94° |
| 14 | 2-$CF_3$,5-Cl | $CF_3$ | |
| 15 | 4-CN | $CF_3$ | |
| 16 | 3-SCN | $CF_3$ | |
| 17 | 2-Cl-4-F | $CF_3$ | |
| 18 | 4-F | $CF_3$ | 214° |
| 19 | 2,4-$F_2$ | $CF_3$ | $n_D^{19}$ : 1.5219 |
| 20 | 4-$CF_2Cl$ | $CF_3$ | 145° |
| 21 | 4-t-But | $CF_3$ | 157° |
| 22 | 3,4-$(OC_2H_5)_2$ | $CF_3$ | 177° |
| 23 | 4-Br | $CF_3$ | 163° |

Tabelle 2

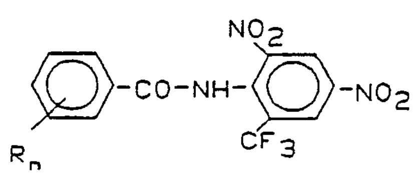

( I )

| Bsp.-Nr. | $R_n$ | Fp |
|---|---|---|
| 24 | $4-CF_3$ | 149° |
| 25 | $2,6-F_2$ | 165° |
| 26 | 4-Cl | 142° |
| 27 | $2,4-Cl_2$ | 158° |
| 28 | $3,4-Cl_2$ | 187° |
| 29 | 3-Cl | 169° |
| 30 | $2-CH_3COO$ | 157° |
| 31 | $3-CF_3$ | 192° |
| 32 | $4-Cl_3CO$ | $n_D^{50}$ : 1,5800 |
| 33 | $2-Cl,4-CF_3$ | 199° |
| 34 | 3-SCN | |
| 35 | 4-CN | |
| 36 | 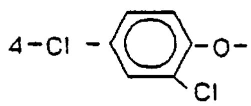 | 191° |
| 37 | 2-Cl,4-F | $n_D^{20}$ : 1,5458 |
| 38 | 4-t-But | 160° |
| 39 | 4-Br | 138° |
| 40 | $3,4-(OC_2H_5)_2$ | 171° |
| 41 | 4-F | 192° |
| 42 | $4-CF_2Cl$ | 110° |

**Tabelle 3**

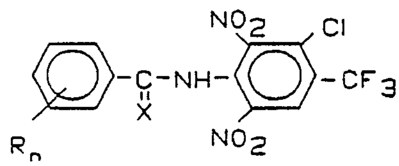

(I)

| Bsp.-Nr. | $R_n$ | X | Fp |
|---|---|---|---|
| 43 | $4-CF_3$ | O | 199° |
| 44 | 4-Cl | O | 211° |
| 45 | 2-Br | O | 246° |
| 46 | 2-Cl,6-F | O | 222° |

23

Tabelle 3 Fortsetzung

Tabelle 3 Fortsetzung

| Bsp.-Nr. | $R_n$ | X | Fp |
|---|---|---|---|
| 47 | 2-Cl | O | 230° |
| 48 | 2,6-$Cl_2$ | O | 230° |
| 49 | 2-$NO_2$ | O | 237° |
| 50 | 2,4-$Cl_2$ | O | 237° |
| 51 | 2,6-$F_2$ | O | 192° |
| 52 | 2-F | O | 178° |
| 53 | 4-$CF_3O$ | O | 204° |
| 54 | 2-$CH_3COO$ | O | 196° |
| 55 | 4-J | O | 220° |
| 56 | 2,3,4,5,6-$F_5$ | O | 183° |
| 57 | 3,4-$Cl_2$ | O | 205° |
| 58 | 3-Cl | O | 197° |
| 59 | 3-$CF_3$ | O | 208° |
| 60 | 4-$Cl_3C$-CO | O | 197° |
| 61 | 4-Cl | S | 82° |
| 62 | 2-OH | O | 170° |
| 63 | 2-Cl,4-$CF_3$ | O | 269° |
| 64 | 4-$CH_3COO$ | O | 160° |
| 65 | 2-Cl | S | 205° |
| 66 | 2-$CONH_2$ 3,4,5,6-$Cl_4$ | O | 300° |
| 67 | 4-F | O | 192° |
| 68 | 2,3-$Cl_2$ | O | |
| 69 | 4-CN | O | 243° |
| 70 | 3-SCN | O | |
| 71 | 4-Cl- 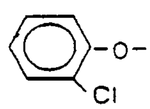 | O | 183° |
| 72 | 2-Cl,4-F | O | $n_D^{20}$ : 1,5398 |
| 73 | 2,4-$F_2$ | O | $n_D^{19}$ : 1.5313 |
| 74 | 4-$CF_2Cl$ | O | 196° |
| 75 | 4-t-But | O | 146° |
| 76 | 3,4-$(OC_2H_5)_2$ | O | 185° |
| 77 | 4-Br | O | 234° |

**Tabelle 4**

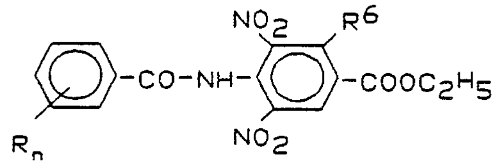

( I )

| Bsp.-Nr. | $R_n$ | $R^6$ | Fp |
|---|---|---|---|
| 78 | 4-$CF_3$ | Cl | 170° |
| 79 | 2,6-$F_2$ | Cl | 210° |
| 80 | 4-Cl | Cl | Wachs |
| 81 | 2,4-$Cl_2$ | Cl | 203° |
| 82 | 3-Cl | Cl | 120° |
| 83 | 2-$CH_3$-COO- | Cl | 152° |
| 84 | 3-$CF_3$ | Cl | 45° |
| 85 | 4-$Cl_3$CO- | Cl | 60° |
| 86 | 2-Cl,4-$CF_3$ | Cl | 282° |
| 87 | 4-$CF_3$ | H | 161° |
| 88 | 2,6-$F_2$ | H | 187° |
| 89 | 4-Cl | H | 101° |
| 90 | 2-Cl,4-F | H | |

**Tabelle 5**

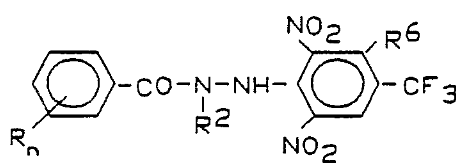

( I )

| Bsp.-Nr. | $R_n$ | $R^2$ | $R^6$ | Fp |
|---|---|---|---|---|
| 91 | 4-$CF_3$ | H | Cl | 217° |
| 92 | 4-$CF_3$ | $CH_3$ | Cl | 56° |
| 93 | 4-$CF_3$ | $CH_3$ | H | Wachs |
| 94 | 4-$CF_3$ | H | H | 225° |

**Tabelle 6**

Verbindung gemäß Formel (I)

X = $NOR^1$, m = O

| Bsp. Nr. | $R_n$ | $R^1$ | $R^2$ | $R^3$ | $R^4$ | $R^5$ | $R^6$ | Fp. |
|---|---|---|---|---|---|---|---|---|
| 95 | 4-Cl | Me | H | $NO_2$ | H | $CF_3$ | H | 134° |
| 96 | 4-Cl | Me | H | $NO_2$ | H | $CF_3$ | Cl | 132° |
| 97 | 4-Cl | Me | H | $CF_3$ | H | $NO_2$ | H | 90° |
| 98 | 4-Cl | Me | H | $CF_3$ | Cl | $NO_2$ | H | |
| 99 | 3-Cl | Me | H | $NO_2$ | H | $CF_3$ | H | 124° |
| 100 | 3-Cl | Me | H | $NO_2$ | H | $CF_3$ | Cl | 120° |
| 101 | 2-Cl | Me | H | $NO_2$ | H | $CF_3$ | Cl | |
| 102 | 4-Cl | $-CH_2-CH=CH_2$ | H | $NO_2$ | H | $CF_3$ | H | 102° |
| 103 | 4-Cl | $-CH_2-CH=CH_2$ | H | $NO_2$ | H | $CF_3$ | Cl | 67° |
| 104 | 4-Cl | $-CH_2-CH=CH_2$ | H | $CF_3$ | H | $NO_2$ | H | |
| 105 | 2,4-$Cl_2$ | Me | H | $NO_2$ | H | $CF_3$ | H | 82° |
| 106 | 2,4-$Cl_2$ | Me | H | $NO_2$ | H | $CF_3$ | Cl | 112° |
| 107 | 3,4-$Cl_2$ | Me | H | $NO_2$ | H | $CF_3$ | H | 70° |
| 108 | 3,4-$Cl_2$ | Me | H | $NO_2$ | H | $CF_3$ | Cl | 103° |
| 109 | 4-F | Me | H | $NO_2$ | H | $CF_3$ | H | 95° |
| 110 | 4-F | Me | H | $NO_2$ | H | $CF_3$ | Cl | 121° |
| 111 | 4-F | Me | H | $CF_3$ | H | $NO_2$ | H | Öl |
| 112 | 2-F | Me | H | $NO_2$ | H | $CF_3$ | Cl | 99° |
| 113 | 2,6-$F_2$ | Me | H | $NO_2$ | H | $CF_3$ | H | 92° |
| 114 | 2,6-$F_2$ | Me | H | $NO_2$ | H | $CF_3$ | Cl | 122° |
| 115 | 2,6-$F_2$ | Me | H | $CF_3$ | H | $NO_2$ | H | 120° |
| 116 | 2,6-$F_2$ | Me | H | $NO_2$ | H | $CO_2Et$ | H | 168° |
| 117 | 2,6-$F_2$ | $-CH_2-CH=CH_2$ | H | $NO_2$ | H | $CF_3$ | H | 72° |
| 118 | 2,6-$F_2$ | $-CH_2-CH=CH_2$ | H | $NO_2$ | H | $CF_3$ | Cl | |
| 119 | 2,6-$F_2$ | $-CH_2-CH=CH_2$ | H | $NO_2$ | H | $CF_3$ | H | 114° |
| 120 | 2,6-$F_2$ | $-CH_2-C{\equiv}CH$ | H | $NO_2$ | H | $CF_3$ | Cl | Öl |
| 121 | 3-Cl,4-F | Me | H | $NO_2$ | H | $CF_3$ | H | |
| 122 | 3-Cl,4-F | Me | H | $NO_2$ | H | $CF_3$ | Cl | |
| 123 | 4-$CF_3$ | Me | H | $NO_2$ | H | $CF_3$ | H | 124° |

**Tabelle 6** Fortsetzung

| Bsp. Nr. | $R_n$ | $R^1$ | $R^2$ | $R^3$ | $R^4$ | $R^5$ | $R^6$ | Fp. |
|---|---|---|---|---|---|---|---|---|
| 124 | 4-$CF_3$ | Me | H | $CF_3$ | H | $NO_2$ | H | 108° |
| 125 | 4-$CF_3$ | Me | H | $CF_3$ | Cl | $NO_2$ | H | |
| 126 | 4-$CF_3$ | Me | H | $NO_2$ | H | $CO_2Et$ | H | 150° |
| 127 | 4-$CF_3$ | Me | H | $CF_3$ | H | $CF_3$ | | |
| 128 | 4-$CF_3$ | Me | $-SCCl_3$ | $NO_2$ | H | $CF_3$ | Cl | |
| 129 | 4-$CF_3$ | Me | H | $NO_2$ | H | CN | H | 130° |
| 130 | 4-$CF_3$ | Me | H | $NO_2$ | H | CN | Cl | 109° |
| 131 | 4-$CF_3$ | Me | H | $NO_2$ | H | $OCF_3$ | H | 63° |
| 132 | 4-$CF_3$ | Me | H | $NO_2$ | Cl | $CF_3$ | Cl | |
| 133 | 4-$CF_3$ | Me | Me | $NO_2$ | H | $CF_3$ | Cl | |
| 134 | 4-$CF_3$ | $-CH_2-CH=CH_2$ | H | $NO_2$ | H | $CF_3$ | H | 82° |
| 135 | 4-$CF_3$ | $-CH_2-CH=CH_2$ | H | $NO_2$ | H | $CF_3$ | Cl | 82° |
| 136 | 4-$CF_3$ | $-CH_2-CH=CH_2$ | H | $CF_3$ | H | $NO_2$ | H | |
| 137 | 4-$CF_3$ | $-COCH_3$ | H | $NO_2$ | H | $CF_3$ | H | 142° |
| 138 | 4-$CF_3$ | $-COCH_3$ | H | $NO_2$ | H | $CF_3$ | Cl | |
| 139 | 4-$CF_3$ | $-COCH_3$ | H | $CF_3$ | H | $NO_2$ | H | |
| 140 | 4-$CF_3$ | $-CH_2-C{\equiv}CH$ | H | $NO_2$ | H | $CF_3$ | H | 106° |
| 141 | 4-$CF_3$ | $-CH_2-C{\equiv}CH$ | H | $NO_2$ | H | $CF_3$ | Cl | 118° |
| 142 | 4-$CF_3$ | $-CH_2-C{\equiv}CH$ | H | $CF_3$ | H | $NO_2$ | H | 114° |
| 143 | 4-$CF_3$ | $-CH_2CN$ | H | $NO_2$ | H | $CF_3$ | H | 106° |
| 144 | 4-$CF_3$ | $-CH_2CN$ | H | $NO_2$ | H | $CF_3$ | Cl | 131° |
| 145 | 4-$CF_3$ | $-CF_2CHFCF_3$ | H | $NO_2$ | H | $CF_3$ | H | |
| 146 | 4-$CF_3$ | $-CF_2CHFCF_3$ | H | $NO_2$ | H | $CF_3$ | Cl | Öl |
| 147 | 4-$CF_3$ | $-CF_2CHFCF_3$ | H | $CF_3$ | H | $NO_2$ | H | |
| 148 | 3-$CF_3$ | Me | H | $NO_2$ | H | $CF_3$ | H | 118° |
| 149 | 3-$CF_3$ | Me | H | $NO_2$ | H | $CF_3$ | Cl | 122° |
| 150 | 3-$CF_3$ | Me | H | $NO_2$ | H | $CO_2Et$ | H | 138° |
| 151 | 3-$CF_3$ | Me | H | $CF_3$ | H | $NO_2$ | H | 105° |
| 152 | 2-Cl,5-$CF_3$ | Me | H | $NO_2$ | H | $CF_3$ | H | 98° |
| 153 | 2-Cl,5-$CF_3$ | Me | H | $NO_2$ | H | $CF_3$ | Cl | 93° |
| 154 | 2-Cl,5-$CF_3$ | Me | H | $CF_3$ | H | $NO_2$ | H | |

Tabelle 7

| Verbindung gemäß Bsp.-Nr. | mit Plasmopara viticola befallene Blattfläche in % bei mg Wirkstoff/Liter Spritzbrühe | | |
|---|---|---|---|
| | 500 | 250 | 125 |
| 43 | 0 | 0 | 0 |
| 3 | 0 | 0 | 0 |
| 44 | 0 | 0 | 0 |
| 55 | 0 | 0 | 0 |
| 57 | 0 | 0 | 0 |
| 58 | 0 | 0 | 0 |
| 59 | 0 | 0 | 0 |
| 31 | 0 | 0 | 0 |
| 95 | 0 | 0 | 0-3 |
| 123 | 0 | 0 | 0 |
| 96 | 0 | 0 | 0 |
| 2 | 0 | 5 | 15 |
| 114 | 0 | 0 | 0 |
| 162 | 0 | 0 | 0 |
| unbehandelte, infizierte Pflanzen | | 100 | |

Tabelle 8

| Verbindung gemäß Bsp.-Nr. | mit Plasmopara viticola befallene Blattfläche in % bei mg Wirkstoff/Liter Spritzbrühe | |
|---|---|---|
| | 500 | 250 |
| 151 | 0 | 0 |
| 124 | 0 | 0 |
| 129 | 0 | 0 |
| unbehandelte, infizierte Pflanzen | | 100 |

**Beispiel 2**

Weizenpflanzen wurden im 3-Blattstadium mit Konidien des Weizenmehltaus (Erysiphe graminis) stark inokuliert und in einem Gewächshaus bei 20° C und einer relativen Luftfeuchte von 90 - 95 % aufgestellt. 3 Tage nach Inokulation wurden die Pflanzen mit den in Tabelle 9 aufgeführten Verbindungen in den angegebenen Wirkstoffkonzentrationen gleichmäßig benetzt. Nach einer Inkubationszeit von 10 Tagen wurden die Pflanzen auf den Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle 9 zusammengefaßt.

Tabelle 9

| Verbindung gemäß Bsp.-Nr. | mit Weizenmehltau befallene Blattfläche in % bei mg Wirkstoff/Liter Spritzbrühe | |
|---|---|---|
| | 500 | 250 |
| 95 | 10 | 10 |
| 123 | 0 | 0-5 |
| unbehandelte, infizierte Pflanzen | | 100 |

**Beispiel 3**

Ca. 6 Wochen alte Apfelsämlinge ("Malus communis") wurden mit den in Tabelle 10 angegebenen Wirkstoffkonzentrationen gleichmäßig benetzt. Nach dem Antrocknen der Spritzbrühe wurden die Pflanzen mit einer Sporensuspension von Venturia inaequalis gleichmäßig tropfnaß benetzt und für 48 Stunden in eine dunkel gehaltene Klimakammer bei einer Temperatur von 20° C und 100 % rel. Luftfeuchte gestellt.

Anschließend wurden die Pflanzen in ein Gewächshaus mit einer Temperatur von 15 - 17° C und ca. 100 % rel. Luftfeuchte gebracht. Ca. 2 Wochen nach der Inokulation erfolgte die Befallauswertung. Der Befallsgrad der Pflanzen mit Apfelschorf wurde in % befallener Blattfläche, bezogen auf unbehandelte, infizierte Pflanzen ausgedrückt und ist in Tabelle 10 wiedergegeben.

Tabelle 10

| Verbindung gemäß Bsp.-Nr. | % Schorfbefall bei mg Wirkstoff/Liter Spritzbrühe | | |
|---|---|---|---|
| | 500 | 250 | 125 |
| 95 | 10 | 10 | 25 |
| 123 | 0 | 0 | 0-3 |
| 2 | 0 | 10 | 10 |
| unbehandelte, infizierte Pflanzen | | 100 | |

**Beispiel 4**

Etwa 5 Wochen alte Reispflanzen der Sorte "Ballila" wurden nach Vorspritzen mit 0,55 %iger Gelatinelösung mit den unten angegebenen Konzentrationen der beanspruchten Verbindungen behandelt. Nach Antrocknen des Spritzbelages wurden die Pflanzen mit einer Sporensuspension von Piricularia oryzae gleichmäßig inokuliert und danach in eine dunkel gehaltene Klimakammer mit einer Temperatur von 25° C und 100 % rel. Luftfeuchte gestellt. Nach 48 Stunden wurden die Reispflanzen in ein Gewächshaus mit einer Temperatur von 25° C und 80 % rel. Luftfeuchte gebracht. Nach 5 Tagen erfolgte die Befallsauswertung. Der Befallsgrad wurde in % befallener Blattfläche im Vergleich zu den unbehandelten, infizierten Kontrollpflanzen ausgedrückt. Die Ergebnisse sind in Tabelle 11 zusammengestellt.

Tabelle 11

| Verbindung gemäß Bsp.-Nr. | mit Piricularia oryzae befallene Blattfläche in % bei mg Wirkstoff/Liter Spritzbrühe | | |
|---|---|---|---|
| | 500 | 250 | 125 |
| 95 | 10 | 10 | 25 |
| 96 | 15 | 15 | 25 |
| 2 | 0-3 | 0-3 | 5 |
| unbehandelte, infizierte Pflanzen | | 100 | |

**Beispiel 5**

Reispflanzen der Sorte "Ballila" wurden im 4 - 5 Blattstadium mit den in Tabelle 12 angegebenen Konzentrationen der beanspruchten Verbindungen behandelt. 24 h nach Antrocknen des Spritzbelags wurden die Reispflanzen in Kunststofftöpfe gestellt und diese bis zum Rand mit Leitungswasser aufgefüllt. Die Inkubation erfolgte, indem eine mit Myzel von Pellicularia sasakii durchwachsene Reisspelzenmischung gleichmäßig auf die Wasseroberfläche verteilt wurde. Danach wurden die Reispflanzen in einem Gewächshaus bei 25 - 30 °C und mindestens 95 % rel. Luftfeuchte für eine Woche inkubiert.

Der Befallsgrad wurde in % befallene Blattscheiden im Vergleich zu den unbehandelten, infizierten Kontrollpflanzen ausgedrückt. Die Ergebnisse sind in Tabelle 12 zusammengestellt.

Tabelle 12

| Verb. gemäß Bsp.-Nr. | mit Pellicularia sasakii befallene Reispflanzen in % bei mg Wirkstoff/Liter Spritzbrühe | | |
|---|---|---|---|
| | 500 | 250 | 125 |
| 95 | 5 | 10 | 10 |
| 123 | 0 | 0 | 0 |
| 96 | 0-3 | 0-3 | 5 |
| 2 | 0 | 0 | 0 |
| 149 | 0 | 0 | 0 |
| 135 | 0 | 0 | 0 |
| 124 | 0 | 0 | 0 |
| unbehandelte, infizierte Pflanzen | | 100 | |

**Beispiel 6**

Weizen der Sorte "Jubilar" wurde im 2-Blattstadium mit wäßrigen Suspensionen der beanspruchten Verbindungen tropfnaß behandelt. Die Anwendungskonzentrationen betrugen 500, 250, 125 und 65 mg Wirkstoff bzw. 500 und 250 mg Wirkstoff pro Liter Spritzbrühe.

Nach dem Antrocknen des Spritzbelages wurden die Pflanzen mit einer wäßrigen Sporensuspension von Puccinia recondita inokuliert. Die Pflanzen wurden für ca. 16 Stunden tropfnaß in eine Klimakammer mit 20 °C und ca. 100 % rel. Luftfeuchtigkeit gestellt. Anschließend wurden die infizierten Pflanzen in ein Gewächshaus mit einer Temperatur von 22 - 25 °C und 50 - 70 % rel. Luftfeuchtigkeit gebracht.

Nach einer Inkubationszeit von ca. 2 Wochen sporuliert der Pilz auf der gesamten Blattoberfläche der nicht behandelten Kontrollpflanzen, so daß eine Befallsauswertung der Versuchspflanzen vorgenommen werden kann. Der Befallsgrad wurde in % befallener Blattfläche im Vergleich zu den unbehandelten, infizierten Kontrollpflanzen ausgedrückt und ist in Tabellen 13 und 14 wiedergegeben.

Tabelle 13

| Verb. gemäß Bsp.-Nr. | mit Puccinia recondita befallene Blattfläche in % bei mg Wirkstoff/Liter Spritzbrühe | | | |
|---|---|---|---|---|
| | 500 | 250 | 125 | 60 |
| 95 | 0 | 0 | 5 | 15 |
| 123 | 0 | 0 | 0 | 0-3 |
| 96 | 0 | 0 | 0 | 0-3 |
| 2 | 0 | 0 | 0 | 0 |
| 151 | 0 | 0 | 0 | 0 |
| 124 | 0 | 0 | 0 | 0 |
| unbehandelte, infizierte Pflanzen | 100 | | | |

Tabelle 14

| Verb. gemäß Bsp.-Nr. | mit Puccina recondita befallene Blattflächen in % bei mg Wirkstoff/Liter Spritzbrühe | |
|---|---|---|
| | 500 | 250 |
| 135 | 0 | 0 |
| 149 | 0 | 0 |
| 148 | 0 | 0 |
| unbehandelte, infizierte Pflanzen | | 100 |

**Beispiel 7**

Mit Bohnenspinnmilben (Tetranychus urticae, Vollpopulation) stark befallene Bohnenpflanzen (Phaseolus v.) wurden mit der wäßrigen Verdünnung eines Emulsionskonzentrates, das 500 ppm des jeweiligen Wirkstoffes enthielt, gespritzt.

Die Mortalität der Milben wurde nach 7 Tagen kontrolliert. 100 % Abtötung wurde mit den Verbindungen gemäß Beispiel 95, 123, 2, 148, 149, 135, 151, 124 und 140 erzielt.

**Beispiel 8**

Mit Kundebohnenblattlaus (Aphis craccivora) stark besetzte Ackerbohnen (Vicia faba) wurden mit wäßrigen Verdünnungen von Spritzpulverkonzentraten mit 1000 ppm Wirkstoffgehalt bis zum Stadium des beginnenden Abtropfens besprüht.

Die Mortalität der Blattläuse wurde nach 3 Tagen bestimmt.

Eine 100 %ige Abtötung konnte mit den Verbindungen gemäß Beispiel 95, 123, 151 und 124 erzielt werden.

**Beispiel 9**

Imagines der Stubenfliege (Musca domestica, 24 h alt) wurden in mit Wirkstoff behandelte Petrischalen eingesetzt. Diese Petrischalen waren zuvor auf der Innenseite des Deckels und des Bodens gleichmäßig mit je 1 ml einer wäßrigen Wirkstoffzubereitung mit einer Konzentration (bezogen auf den Wirkstoff) von 250 ppm in Wasser behandelt worden. Nach dem Abtrocknen wurden die Tiere eingesetzt, die Schalen verschlossen und bei 22 °C 3 h aufbewahrt. Nach dieser Zeit wurden die toten Fliegen gezählt und die Mortalität festgestellt.

Es zeigte sich, daß die Verbindungen gemäß Bsp. 96, 2, 114, 151, 124, 164, 129 und 113 in der

genannten Dosierung zu 100 % Mortalität hervorrufen.

**Beispiel 10**

Die Bodeninnenseiten von mit künstlichem Nährmedium beschichteten Petrischalen wurden nach dem Erstarren des Futterbreis mit jeweils 3 ml einer 250 ppm an Wirkstoff enthaltenden, wäßrigen Emulsion besprüht. Nach Abtrocknen des Spritzbelages und Einsetzen von 10 Larven des Gemeinen Baumwollwurmes (Prodenia litura) wurden die Schalen 7 Tage bei 21 °C aufbewahrt und der Wirkungsgrad der jeweiligen Verbindungen (ausgedrückt in % Mortalität) bestimmt. Die Verbindungen 154, 124 und 129 ergaben in diesem Test eine Wirksamkeit von jeweils 100 %.

**Beispiel 11**

Blätter der Bohne (Phaseolus vulgaris, wurden mit einer wäßrigen Emulsion der Verbindungen in einer Konzentration von 250 ppm (bezogen auf Wirkstoff) besprüht und zu gleich behandelten Larven des Mexikanischen Bohnenkäfers (Epilachna varivestis) in Beobachtungskäfige gestellt. Die Verbindungen laut 151 und 124 zeigten nach 48 h eine 100 %ige Abtötung.

**Ansprüche**

1. Verbindungen der Formel I

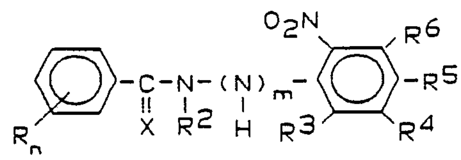

(I),

worin

X O, S oder $NOR^1$,

R Wasserstoff, Halogen, CN, SCN, $NO_2$, OH, Carbamoyl, $(C_1-C_4)$-Alkyl, $(C_1-C_4)$-Alkoxy, $(C_1-C_4)$-Thioalkyl, die. ganz oder teilweise durch Halogen substituiert sein können, Phenyl, Thiophenyl oder Phenoxy. die durch 1 oder 2 Halogenatome oder eine $CF_3$-Gruppe substituiert sein können, oder $(C_1-C_4)$-Acyl,

$R^1$ Wasserstoff, $(C_1-C_4)$-Alkyl, $(C_2-C_4)$-Alkenyl, $(C_2-C_4)$-Alkinyl, die ganz oder teilweise durch Halogen substituiert sein können, $(C_1-C_4)$-Acyl, Cyanomethyl,

$R^2$ Wasserstoff, $(C_1-C_4)$-Alkyl oder $S-CCl_3$,

$R^3$,$R^5$ jeweils unabhängig voneinander der $NO_2$, Halogen, CN, COOH, $CONH_2$, $(C_1-C_4)$-Alkyl oder $(C_1-C_4)$-Alkoxy, die ganz oder teilweise durch Halogen substituiert sein können, $(C_1-C_4)$-Alkoxycarbonyl, $CONR^7R^8$,

$R^4$ Wasserstoff oder Halogen,

$R^6$ Wasserstoff, Halogen, $(C_1-C_4)$-Alkoxy oder Phenoxy, $R^7$,$R^8$ $(C_1-C_4)$-Alkyl,

m 0 oder 1 und

n 0 - 5

bedeuten, mit der Maßgabe, daß im Fall X = $NOR^1$ m = 0 sein muß, sowie deren in den Landwirtschaft einsetzbaren Salze.

2. Verbindungen der Formel I von Anspruch 1, worin R = Halogen oder $CF_3$, n = 1 oder 2, X = Sauerstoff oder $NOR^1$, $R^1$ = $(C_1-C_4)$-Alkyl, $R^2$ = H, $R^3$ = $NO_2$ oder $CF_3$, $R^4$ = H, $R^5$ = $NO_2$, $CF_3$ oder CN, $R^6$ = Wasserstoff oder Chlor und m = 0 bedeuten sowie deren in der Landwirtschaft einsetzbaren Salze.

3. Verfahren zur Herstellung von Verbindungen der Formel I gemäß Anspruch 1 oder 2, dadurch gekennzeichnet, daß man

a) eine Verbindung der Formel (II)

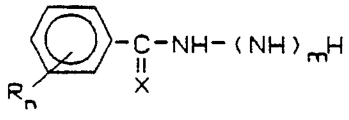

(II),

worin R, X, n und m die Bedeutungen wie in Formel I besitzen, mit einer Verbindung der Formel (III)

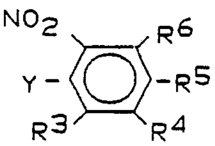

(III),

worin $R^3$, $R^4$, $R^5$ und $R^6$ die Bedeutungen wie in Formel I besitzen und Y Halogen bedeutet, in Gegenwart einer Base umsetzt. oder

b) eine Verbindung der Formel (IV)

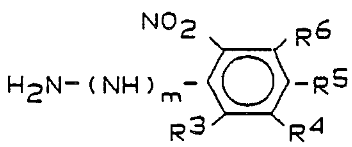

(IV),

worin m, $R^3$, $R^4$, $R^5$ und $R^6$ die Bedeutungen wie in Formel 1 besitzen, mit einer Verbindung der Formel (V)

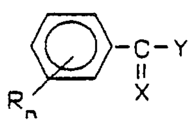

(V),

worin R, n und X die Bedeutungen wie in Formel I besitzen und Y Halogen bedeutet, umsetzt.

4. Insektizide, fungizide, akarizide oder nematozide Mittel, dadurch gekennzeichnet, daß sie eine wirksame Menge einer Verbindung der Formel I enthalten.

5. Verwendung der Verbindungen der Formel I zur Bekämpfung von Akariden, Schadinsekten, Schadpilzen oder Nematoden.

6. Verfahren zur Bekämpfung von Akariden, Schadinsekten, Schadpilzen oder Nematoden, dadurch gekennzeichnet, daß man auf diese oder die von ihnen befallenen Pflanzen, Flächen oder Substrate eine wirksame Menge einer Verbindung der Formel I appliziert.

Patentansprüche für folgenden Vertragsstaat: ES

1 Verfahren zur Herstellung von Verbindungen der Formel I

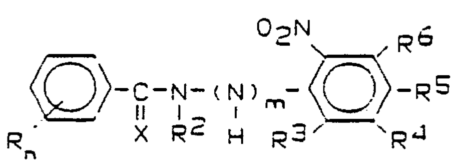

(I),

worin

X O, S oder $NOR^1$,

R Wasserstoff, Halogen, CN, SCN, $NO_2$, OH, Carbamoyl, $(C_1-C_4)$-Alkyl, $(C_1-C_4)$-Alkoxy, $(C_1-C_4)$-Thioalkyl, die ganz oder teilweise durch Halogen substituiert sein können, Phenyl, Thiophenyl oder Phenoxy, die durch 1 oder 2 Halogenatome oder eine $CF_3$-Gruppe substituiert sein können, oder $(C_1-C_4)$ Acyl,

$R^1$ Wasserstoff, $(C_1-C_4)$-Alkyl, $(C_2-C_4)$-Alkenyl, $(C_2-C_4)$-Alkinyl, die ganz oder teilweise durch Halogen substituiert sein können, $(C_1-C_4)$-Acyl, Cyanomethyl,

$R^2$ Wasserstoff, $(C_1-C_4)$-Alkyl oder $S-CCl_3$,

$R^3$,$R^5$ jeweils unabhängig voneinander $NO_2$, Halogen, CN, COOH, $CONH_2$, $(C_1-C_4)$-Alkyl oder $(C_1-C_4)$-Alkoxy, die ganz oder teilweise durch Halogen substituiert sein können, $(C_1-C_4)$-Alkoxycarbonyl, $CONR^7R^8$,

$R^4$ Wasserstoff oder Halogen,

$R^6$ Wasserstoff, Halogen, $(C_1-C_4)$-Alkoxy oder Phenoxy,

$R^7$,$R^8$ $(C_1-C_4)$-Alkyl,

m 0 oder 1 und

n 0 - 5

bedeuten, mit der Maßgabe, daß im Fall X = $NOR^1$ m = 0 sein muß, sowie deren in den Landwirtschaft einsetzbaren Salze, dadurch gekennzeichnet, daß man

a) eine Verbindung der Formel (II)

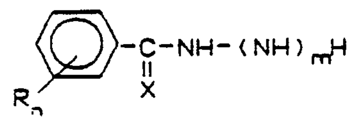

(II),

worin R, X, n und m die Bedeutungen wie in Formel I besitzen, mit einer Verbindung der Formel (III)

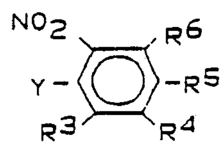

(III),

worin $R^3$, $R^4$, $R^5$ und $R^6$ die Bedeutungen wie in Formel I besitzen und Y Halogen bedeutet, in Gegenwart einer Base umsetzt, oder

b) eine Verbindung der Formel (IV)

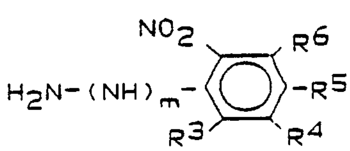

(IV),

worin m, $R^3$, $R^4$, $R^5$ und $R^6$ die Bedeutungen wie in Formel I besitzen, mit einer Verbindung der Formel (V)

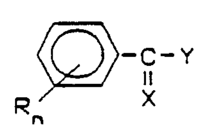

(V),

worin R, n und X die Bedeutungen wie in Formel I besitzen und Y Halogen bedeutet, umsetzt.

2. Verfahren gemäß Anspruch 1, dadurch gekennzeichnet, daß in Formel I R = Halogen oder $CF_3$, n = 1 oder 2, X = Sauerstoff oder $NOR^1$, $R^1$ = $(C_1-C_4)$-Alkyl, $R^2$ = H, $R^3$ = $NO_2$ oder $CF_3$, $R^4$ = H, $R^5$ = $NO_2$, $CF_3$ oder CN, $R^6$ = Wasserstoff oder Chlor und m = 0 bedeuten, sowie deren in der Landwirtschaft einsetzbaren Salze.

3. Insektizide, fungizide, akarizide oder nematozide Mittel, dadurch gekennzeichnet, daß sie eine wirksame Menge einer Verbindung der Formel I enthalten.

4. Verwendung der Verbindungen der Formel I zur Bekämpfung von Akariden, Schadinsekten, Schadpilzen oder Nematoden.

5. Verfahren zur Bekämpfung von Akariden, Schadinsekten, Schadpilzen oder Nematoden, dadurch gekennzeichnet, daß man auf diese oder die von ihnen befallenen Pflanzen, Flächen oder Substrate eine wirksame Menge einer Verbindung der Formel I appliziert.